(19) Europäisches Patentamt / European Patent Office / Office européen des brevets

(11) **EP 3 898 895 B1**

(12) **EUROPEAN PATENT SPECIFICATION**

(45) Date of publication and mention of the grant of the patent:
**01.05.2024 Bulletin 2024/18**

(21) Application number: **19827770.9**

(22) Date of filing: **16.12.2019**

(51) International Patent Classification (IPC):
$C07C\ 4/04^{(2006.01)}$      $C07C\ 5/05^{(2006.01)}$
$C10G\ 3/00^{(2006.01)}$      $C10G\ 55/04^{(2006.01)}$
$C10G\ 57/00^{(2006.01)}$     $C07C\ 41/06^{(2006.01)}$
$C10G\ 69/06^{(2006.01)}$     $C10G\ 9/00^{(2006.01)}$
$C10G\ 45/58^{(2006.01)}$     $C07C\ 2/56^{(2006.01)}$

(52) Cooperative Patent Classification (CPC):
(C-Sets available)
C10G 3/50; C07C 2/56; C07C 4/04; C07C 5/05;
C07C 41/06; C10G 9/00; C10G 45/58; C10G 55/04;
C10G 57/005; C10G 69/06; Y02P 20/582;
Y02P 30/20                                    (Cont.)

(86) International application number:
**PCT/FI2019/050894**

(87) International publication number:
**WO 2020/128156 (25.06.2020 Gazette 2020/26)**

(54) **METHOD TO PRODUCE HIGH QUALITY COMPONENTS FROM RENEWABLE RAW MATERIAL**

VERFAHREN ZUR HERSTELLUNG VON HOCHWERTIGEN KOMPONENTEN AUS NACHWACHSENDEN ROHSTOFFEN

PROCÉDÉ DE PRODUCTION DE COMPOSANTS DE QUALITÉ SUPÉRIEURE À PARTIR DE MATIÈRE PREMIÈRE RENOUVELABLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.12.2018 FI 20186098**

(43) Date of publication of application:
**27.10.2021 Bulletin 2021/43**

(73) Proprietor: **Neste Oyj**
**02150 Espoo (FI)**

(72) Inventors:
• **KARVO, Anna**
 **06101 Porvoo (FI)**
• **RÄMÖ, Virpi**
 **06101 Porvoo (FI)**
• **KIISKI, Ulla**
 **06101 Porvoo (FI)**
• **ROUHIAINEN, Maija**
 **06101 Porvoo (FI)**

(74) Representative: **TBK**
**Bavariaring 4-6**
**80336 München (DE)**

(56) References cited:
CN-A- 107 056 570      US-A- 3 816 294
US-A1- 2002 087 040    US-A1- 2016 326 079
US-A1- 2018 179 458    US-A1- 2018 282 632

• ZHIWEI CHEN ET AL: "Mechanism of byproducts formation in the isobutane/butene alkylation on HY zeolites", RSC ADVANCES, vol. 8, no. 7, 1 January 2018 (2018-01-01) , pages 3392-3398, XP055664531, DOI: 10.1039/C7RA12629H

(52)   Cooperative Patent Classification (CPC): (Cont.)

C-Sets
**C07C 2/56, C07C 9/21;**
**C07C 4/04, C07C 11/08;**
**C07C 4/04, C07C 11/09;**
**C07C 5/05, C07C 11/08;**
**C07C 41/06, C07C 43/046**

## Description

### Technical Field

The present invention relates to a method of producing high quality components from renewable raw material. Specifically, the invention relates to the production of renewable materials which can be employed as high-quality chemicals and/or as high quality drop-in gasoline components, more specifically as high-octane components in gasoline fuel. Further, the invention relates to drop-in gasoline components and to polymers obtainable by the method of the invention.

### Background of the Invention

Production of fuel components from biomass is of increasing interests since they are produced from a sustainable source of organic compounds.

However, while several routes for the production of diesel components having reasonable properties from renewable sources are available in the art, there is still demand for easily accessible renewable gasoline components which can be blended in high amounts and which do not deteriorate the properties of the fuel. For example, according to current European standard (EN228), ethanol can be blended into regular fuel in an amount of at most 10 vol-%. However, a blend of conventional fuel with ethanol (roughly up to 50 vol-%) shows a significant increase of vapour pressure (DVPE; dry vapour pressure equivalent). Similarly, products produced from renewable sources, such as fats and oils, by hydrogenation and optional isomerization usually boil in the diesel fuel range and are of low value as gasoline fuel components. On the other hand, the production process of such diesel fuel components may comprise cracking side reactions which usually provides hydrocarbons in the naphtha range, specifically C5 to C10 hydrocarbons. While this naphtha fulfils the requirements of gasoline as regards boiling point ranges, the allowable blending amount is rather low due to the generally poor octane number.

Similarly, hydrocarbon components derived from other biological raw material can be used as blend components in fuel. For example, EP 2643442 B1 discloses a process for purifying tall oil material and indicates that one of the resulting fractions may be hydrogenated and used as a gasoline, naphtha, jet or diesel fuel component.

US 2012 / 0142982 A1 discloses the production of bio-monomers and/or gasoline components by steam cracking using a complex mixture of fatty acids or triglycerides derived from naturally occurring oils and fats as a steam cracker feed. The gasoline fraction is obtained after removal of the C1 to C4 reaction products.

Nevertheless, there is still need for renewable drop-in gasoline fuel components having high octane number which thus can be blended in any desired amount.

Further, US 2018/282632 A1 discloses a method of producing biohydrocarbons, which includes providing an isomeric raw material obtained from a bio-renewable feedstock, and containing at least 65 wt. % iso-paraffins, and thermally cracking the isomeric raw material to produce biohydrocarbons at a temperature of at most 825° C. The biohydrocarbons can further be polymerized to obtain bio-polymers. US 2018/179458 A1 discloses a method of producing biohydrocarbons. Further, the invention relates to biohydrocarbons obtainable by the methods of the invention and to a method of producing polymers. CN 107056570 A discloses a process for an isooctane-refined alkylated raw material gas. US 2002/087040 A1 discloses a process for the production of hydrocarbons with a high octane number starting from mixtures essentially consisting of n-butane and isobutene, and comprising a skeleton isomerization section, a dehydrogenation section of paraffins, a selective hydrogenation section of butadiene, two conversion sections of olefins, in which the isobutene is firstly selectively transformed by means of dimerization and/or etherification, followed by the linear butenes by means of alkylation, in order to obtain, by joining the products of the two conversion sections, a product. US 2016/326079 A1 discloses methods for producing methyl t-butyl ether (MTBE) by reacting an isobutene-containing stream with methanol in an MTBE production unit. US 3816294 A discloses a method of preparing a product stream containing alkylate gasoline from a charge stream containing paraffinic components. Zhiwei C. et al, disclose "Mechanism of byproducts formation in the isobutane/butene alkylation on HY zeolites", RSC ADVANCES, vol. 8, no. 7, pages 3392 - 3398.

### Summary of Invention

The present invention was made in view of the above-mentioned problems and it is an object of the present invention to provide an improved process for producing renewable drop-in gasoline components.

In brief, the present invention relates to a method for producing renewable component(s), the method comprising:

a provision step of providing an isomeric raw material originating from a renewable source, wherein the isomeric raw material contains at least 60 wt.-% iso-paraffins and the iso-paraffins of the isomeric raw material contain more than 30 wt.-% multi-branched iso-paraffins, wherein the amounts of iso-paraffins and multi-branched iso-paraffins are determined relative to the total paraffin content in the isomeric raw material,

a cracking step of thermally cracking the isomeric raw material to produce a biohydrocarbon mixture containing C4 olefins, and

a reaction step of reacting at least a part of the C4 olefins to produce the renewable component(s).

The renewable component(s) may be drop-in gasoline component(s) having a research octane number (RON) of at least 90.

The renewable component(s) may be bio-monomer(s) or bio-polymer(s).

The bio-monomer(s) or bio-polymer(s) may be at least one selected from the group consisting of butyl rubber, methyl methacrylate, polymethyl methacrylate, polyisobutylene, substituted phenol, and polybutene.

Preferably, the mixture containing C4 olefins contains at least isobutene and the reaction step of reacting at least a part of the C4 olefins is a step of reacting at least a part of the isobutene to produce the renewable component(s).

The isomeric raw material may contain at least 70 wt.-%, preferably at least 75 wt.-%, at least 80 wt.-%, at least 83 wt.-%, at least 85 wt.-%, at least 90 wt.-%, or at least 95 wt.-% iso-paraffins.

The isomeric raw material preferably contains 60 to 99 wt.-% iso-paraffins, or 60 to 98 wt.-% iso-paraffins.

The thermal cracking in the cracking step is preferably conducted at a temperature (coil outlet temperature COT) in the range of 720°C to 880°C.

The thermal cracking in the cracking step may be conducted at a temperature (coil outlet temperature COT) of at least 720°C, preferably at least 740°C, at least 760°C, or at least 780°C

The thermal cracking in the cracking step may be conducted at a temperature (coil outlet temperature COT) of at most 880°C, preferably at most 860°C, at most 850°C, or at most 840°C.

The provision step preferably comprises an isomerization step of subjecting at least straight chain alkanes in a hydrocarbon material originating from the renewable source to an isomerization treatment to prepare the isomeric raw material.

The provision step preferably comprises a deoxygenation step of deoxygenating a renewable feedstock originating from the renewable source and optionally a subsequent isomerization step to prepare the isomeric raw material.

The deoxygenation step may be a hydrotreatment step, preferably a hydrodeoxygenation step.

Preferably, the renewable source comprises at least one of vegetable oil, vegetable fat, animal oil and animal fat and is subjected to hydrotreatment and optionally to isomerization to prepare the isomeric raw material.

Preferably, the isomeric raw material comprises at least one of a diesel range fraction and a naphtha range fraction and at least the diesel range fraction and/or the naphtha range fraction is subjected to thermal cracking.

As the case may be, only the diesel range fraction and/or the naphtha range fraction, preferably only the diesel range fraction, is subjected to thermal cracking.

The isomeric raw material preferably contains at most 1 wt.-% oxygen based on all elements constituting the isomeric raw material, as determined by elemental analysis.

The thermal cracking in the cracking step preferably comprises steam cracking.

The steam cracking is preferably performed at a flow rate ratio between water and the isomeric raw material ($H_2O$ flow rate [kg/h] / iso-HC flow rate [kg/h]) of 0.05 to 1.10.

The flow rate ratio between water and the isomeric raw material may be at least 0.10, preferably at least 0.15, at least 0.20, or at least 0.25.

The flow rate ratio between water and the isomeric raw material is preferably at most 1.00, preferably at most 0.80, at most 0.60, or at most 0.50.

The biohydrocarbon mixture preferably comprises at least 8.0 wt.-% C4 olefins, relative to all organic components.

The biohydrocarbon mixture may comprise at least 10.0 wt.-%, preferably at least 12.0 wt.-%, at least 14.0 wt.-%, or at least 15.0 wt.-% C4 olefins, relative to all organic components.

The reaction step may comprise a step of subjecting at least one of the C4 olefins, preferably at least one of 1-butene, (Z)-2-butene and (E)-2-butene, to an alkylation reaction.

The alkylation reaction may comprise a reaction between the at least one C4 olefin and a C4 or C5 alkane, preferably an isoalkane.

The alkylation reaction preferably comprises a reaction between the at least one of C4 olefin and isobutane to produce isooctane.

The reaction step may further comprise a step of subjecting at least butadiene contained in the C4 olefins to selective hydrogenation to produce a butene (monoene) and employing the thus produced butene as the at least one C4-olefin alone or in admixture with one or more of the other C4 olefins (excluding butadiene).

The iso-paraffins of the isomeric raw material preferably contain more than 40 wt.-% multi-branched iso-paraffins.

The iso-paraffins of the isomeric raw material preferably contain predominantly multi-branched iso-paraffins.

The iso-paraffins of the isomeric raw material preferably contain more than 50 wt.-% multi-branched iso-paraffins.

The iso-paraffins of the isomeric raw material preferably contain more than 55 wt.-%, even more preferably more than 60 wt.-% multi-branched iso-paraffins.

The multiple branched iso-paraffins may be iso-paraffins having at least dimethyl substitution, and are preferably dimethyl, trimethyl, or higher (methyl) substituted iso-paraffins.

The isomeric raw material is preferably a fraction comprising 50 wt.-% or more of C10-C20 hydrocarbons (based on the organic components).

The isomeric raw material may be a fraction comprising 75 wt.-% or more of C10-C20 hydrocarbons, preferably 90 wt.-% or more of C10-C20 hydrocarbons (based on the organic components).

The content of even-numbered hydrocarbons in the C10-C20 range in the fraction is preferably more than 50 wt.-%.

The fraction may contain

1.0 wt.-% or less, preferably 0.5 wt.-% or less, more preferably 0.2 wt.-% or less aromatics,

less than 2.0, preferably 1.0 wt.-% or less, more preferably 0.5 wt.-% or less of olefins,

5.0 wt.-% or less, preferably 2.0 wt.-% or less naphthenes,

1.0 wt.-% or less, preferably 0.2 wt.-% or less, more preferably 0.1 wt.-% or less oxygenated compounds, and

1.0 wt.-% or less, preferably 0.5 wt.-% or less, more preferably 0.2 wt.-% or less heteroatom-containing compounds.

The reaction step preferably comprises a step of subjecting at least a part of isobutene contained in the C4 olefins to a etherification with a C1 to C3 alcohol to produce a C1 to C3 alkyl tert-butyl ether.

The reaction step preferably comprises a step of subjecting at least a part of isobutene contained in the C4 olefins to a etherification with methanol and/or ethanol to produce methyl t-butyl ether (MTBE) and/or ethyl t-butyl ether (ETBE).

**Brief Description of Drawings**

Fig. 1 shows a schematic picture of a laboratory scale steam cracking setup used in some of the Examples illustrating embodiments of the present invention;

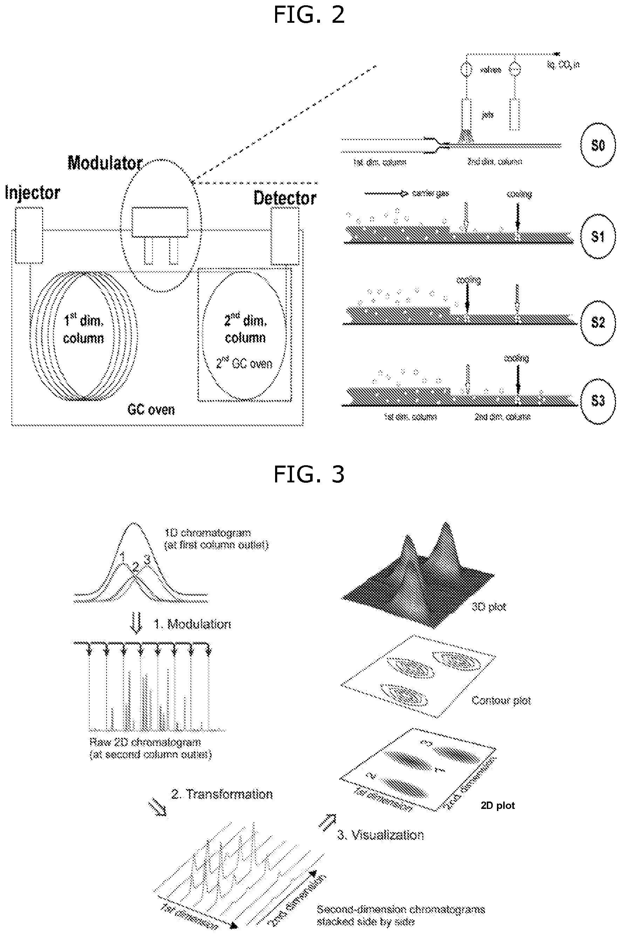

Figs. 2 and 3 show a schematic diagram of the effluent analysis performed in some of the Examples illustrating embodiments of the present invention;

Fig. 4 shows reference components for GCxGC analysis.

**Detailed description of the invention**

The present invention relates to a method of producing renewable component(s) (specifically high-quality components from a raw material originating from a renewable source), the method comprising thermally cracking an iso-paraffin composition (in the following: isomeric raw material) having a high content (at least 60 wt.-%) of iso-paraffins. The iso-paraffin composition may be obtained by isomerization of a hydrocarbon material derived from a renewable feedstock.

In general, the present invention relates to a method of producing high-quality components derived from a renewable feedstock, thus contributing to environmental sustainability of industry depending on petrochemical products, specifically polymer industry and fuel industry.

The present invention provides a method for producing renewable component(s), the method comprising a provision step of providing an isomeric raw material originating from a renewable source, wherein the isomeric raw material contains at least 60 wt.-% iso-paraffins, a cracking step of thermally cracking the isomeric raw material to produce a biohydrocarbon mixture containing C4 olefins, and a reaction step of reacting at least a part of the C4 olefins to produce the renewable component(s).

The provision step of the method may comprise a preparation step of preparing a hydrocarbon material obtainable from a renewable feedstock, and an isomerization step of subjecting at least the straight chain hydrocarbons in the hydrocarbon material to an isomerization treatment to prepare the isomeric raw material.

Using the method of the present invention, it is possible to convert a renewable feedstock into a biohydrocarbon mixture containing a high amount of C4 olefins which is further processed to produce high-quality components for further use in e.g. fuel or polymer industry. As a matter of course, other components of the biohydrocarbon mixture are useful as well, e.g. as solvents, binders, modifiers or in fuel industry.

The term "hydrocarbon material", as used herein refers to a hydrocarbon compound, or a mixture of hydrocarbon compounds, derived from a renewable feedstock (or a renewable source). The "hydrocarbon material" is usually obtained by deoxygenating a renewable feedstock (the renewable feedstock originating from a renewable source), and in this case the hydrocarbon material contains oxygen-containing compounds only as impurities, usually in an amount of 3.0 wt.-% or less, preferably 2.0 wt.-% or less, 1.5 wt.-% or less, 1.0 wt.-% or less, 0.8 wt.-% or less, 0.5 wt.-% or less, or 0.1 wt.-% or less. Generally, it is preferable that the hydrocarbon material contains oxygen-containing compounds in an amount of 6.0 wt.-% or less, preferably 4.0 wt.-% or less, 3.0 wt.-% or less, 2.0 wt.-% or less, 1.5 wt.-% or less, 1.0 wt.-% or less, 0.8 wt.-% or less, 0.5 wt.-% or less, or 0.1 wt.-% or less.

The term "biohydrocarbon mixture" in the present invention refers to the (hydrocarbon) product resulting from the cracking step, optionally after purification and/or separation. The "biohydrocarbon mixture" is a mixture of hydrocarbons and may contain other compounds (such as oxygenates and heteroatom-containing compounds) as impurities.

As used herein "isomeric raw material" refers to a composition derived from a renewable feedstock or renewable source or sources, the composition mainly containing paraffins, and comprising iso-paraffins. According to the invention, the content of iso-paraffins in the isomeric raw material is at least 60.0 wt.-%.

As used herein, the term "diesel range fraction" refers to a fraction or composition having a boiling point ranging from 180 to 360 °C measured according to EN ISO 3405:2011. As used herein, the term "naphtha range fraction" refers to a fraction or composition having a boiling point ranging from 30 to 180°C measured according to EN-ISO-3405 (2011).

As used herein, "paraffin content" is the combined wt.-% amounts of n-paraffins and iso-paraffins. As used herein, "iso-paraffin content" is the wt.-% amounts of branched paraffins. The term "branched paraffins" (or "branched iso-paraffins") refers to both monobranched iso-paraffins and multiple branched iso-paraffins.

The "isomerization degree" is used herein to refer to the amount of isomerized paraffins relative to total paraffin content in a composition. Said amount may be expressed in wt.-%.

**Isomeric raw material**

The isomeric raw material of the present invention contains iso-paraffins (i-paraffins) and may contain normal paraffins (n-paraffins). The isomeric raw material has a high paraffin content of at least 60 wt.-% in order to ensure achieving a high content of C4 olefins in the cracking step. The isomeric raw material comprises preferably at least 90 wt.-% paraffins. More preferably, the isomeric raw material comprises at least 95 wt.-% paraffins. Most preferably, the isomeric raw material contains at least 99 wt.-% paraffins. Components other than paraffins, such as other hydrocarbons (e.g. aromatics, naphthenes or olefins), oxygenated organic compounds (containing one or more oxygen atom) or heteroatom-containing organic components (containing one or more atom other than carbon, hydrogen or oxygen) may be present as well but their content is preferably low. Specifically, the total content of oxygenated organic compounds and heteroatom-containing organic components is preferably less than 3.0 wt.-%.

The iso-paraffins of the isomeric raw material may comprise multiple branched iso-paraffins and monobranched iso-paraffins and preferably comprises both. Monobranched iso-paraffins are paraffins (non-cyclic alkanes) having one sidechain or branch. Multiple branched iso-paraffins, also referred to as multi-branched iso-paraffins, are paraffins (non-cyclic alkanes) having at least two sidechains or branches. Said multiple branched iso-paraffins may have two, three, or more sidechains, or branches. In a preferred embodiment, the monobranched iso-paraffins are monomethyl substituted iso-paraffins, i.e. iso-paraffins having one methyl sidechain or branch. The multiple branched iso-paraffins are preferably at least dimethyl substituted iso-paraffins, preferably dimethyl, trimethyl, or higher (methyl) substituted iso-paraffins, i.e. non-cyclic dimethyl, trimethyl, or higher (methyl) substituted alkanes.

The combined yield of C4 olefins from the thermal cracking step is promoted by using an isomeric raw material containing at least 60 wt.-% iso-paraffins. wt.-%

In the present invention, the content of the iso-paraffins in the isomeric raw material is at least 60 wt.-%. Employing an isomeric raw material having a high content of iso-paraffins ensures good yield of C4 olefins in the cracking step and thus enables efficient production of the high-quality chemicals (components) of the present invention.

In the present invention, the iso-paraffins comprises multi-branched iso-paraffins. The iso-paraffins contain >30 wt.-%, preferably >40 wt.%, more preferably >50 wt.-%, even more preferably >55 wt.-%, or >60 wt.-% multi-branched iso-paraffins. It is further preferred that the iso-paraffins contain predominantly (>50 wt.-%, preferably >55 wt.-%, more preferably >60 wt.-%) multi-branched iso-paraffins. It has been found that increasing the amount of multi-branched iso-paraffins promotes the formation of C4 olefins in the thermal cracking process.

The remainder of the paraffins in the isomeric raw material are n-paraffins. In other words, the paraffins of the isomeric raw material that are not iso-paraffins are n-paraffins.

Without being bound by any theory, it is believed that during the isomerization the substitution, particularly monomethyl substitution, is most likely in the second carbon atom in the linear carbon chain, and that the substitution of the second carbon promotes the formation of propene because the tertiary carbon bonds are most susceptible for cracking. Linear n-paraffins tend to crack to ethene molecules whereas high branching, i.e. multi-branched iso-paraffins, promotes the formation of propene but also of isobutene and other heavier components. Mono branching has been observed to promote the propene yield while the formation of C4+ hydrocarbons stays low. Therefore, it is preferably in the present invention that the content of multi-branched iso-paraffins in the isomeric raw material be high.

In the present invention, the total (wt.-%) amount of paraffins in the isomeric raw material is determined relative to all organic material which is fed to the cracker (relative to all the organic material in the isomeric raw material). The (wt.-%) amounts of iso-paraffins, n-paraffins, monobranched iso-paraffins, and multiple branched iso-paraffins are determined relative to the total paraffin content in the isomeric raw material.

The (wt.-%) amounts of iso-paraffins (monobranched iso-paraffins and multiple branched iso-paraffins) and n-paraffins may be determined using GC-FID analysis, as explained in the Examples, or by any other suitable method. In general, any isomeric raw material as defined above can be used in the present invention. Nevertheless, a specific paraffin fraction is to be highlighted. This Fraction comprises more than 50 wt.-%, preferably 75 wt.-% or more, more preferably 90 wt.-% or more of C10-C20 hydrocarbons (based on the organic components). The content of even-numbered hydrocarbons in the C10-C20 range (i.e. C10, C12, C14, C16, C18, and C20) is preferably more than 50 wt.-%. The fraction contains 1.0 wt.-% or less, preferably 0.5 wt.-% or less, more preferably 0.2 wt.-% or less aromatics, and less than 2.0, preferably 1.0 wt.-% or less, more preferably 0.5 wt.-% or less of olefins, 5.0 wt.-% or less, preferably 2.0 wt.-% or less naphthenes (cyclic alkanes), 1.0 wt.-% or less, preferably 0.2 wt.-% or less, more preferably 0.1 wt.-% or less oxygenated compounds and 1.0 wt.-% or less, preferably 0.5 wt.-% or less, more preferably 0.2 wt.-% or less heteroatom-containing compounds. A low amount of aromatics, olefins, and naphthenes in the thermal cracking feed improves the product distribution of the cracking process. In other words, the smaller the amount (wt.-%) of aromatics, olefins, and naphthenes in the thermal cracking feed, the better the product distribution of the cracking process. "Better product distribution" refers in this context to a product distribution containing more high value products.

In any case, the isomeric raw material preferably contains at most 1 wt.-% oxygen based on all elements constituting the isomeric raw material, as determined by elemental analysis. A low oxygen content of the isomeric raw material (the organic material fed to thermal cracking) allows carrying out the cracking in a more controlled manner, thus resulting in a more favourable product distribution.

The isomeric raw material may be a blend of materials originating from the renewable source and materials of fossil origin, such as fossil naphtha, but preferably contains at least 20 wt.-% of renewable components, more preferably at least 50 wt.-% or at least 80 wt.-% and may be a fully (100%) renewable isomeric raw material.

Carbon atoms of renewable origin comprise a higher number of $^{14}$C isotopes compared to carbon atoms of fossil origin. Therefore, it is possible to distinguish between a renewable (isomeric) paraffin composition and paraffin compositions derived from fossil sources by analysing the ratio of $^{12}$C and $^{14}$C isotopes. Thus, a particular ratio of said isotopes can be used as a "tag" to identify a renewable (isomeric) paraffin composition and differentiate it from non-renewable paraffin compositions. The isotope ratio does not change in the course of chemical reactions.

## Renewable feedstock

In the present invention, the isomeric raw material may be derived from a renewable feedstock as a renewable source. The isomeric raw material may further be derived from a renewable feedstock which in turn is derived from a renewable source.

The renewable feedstock may be the renewable source (i.e. both materials may be the same) or the renewable feedstock may be derived from the renewable source by purification. Further, the renewable feedstock may be a blend of materials originating from the renewable source and materials of fossil origin, such as fossil naphtha, but preferably contains at least 20 wt.-% of renewable components, more preferably at least 50 wt.-% or at least 80 wt.-% and may be a fully (100%) renewable feedstock. In this respect, the renewable source may be one or more renewable sources, i.e. the renewable feedstock may comprise materials originating from different renewable sources, which are herein simply referred to as "renewable source".

The renewable feedstock may be derived from any renewable origin, such as materials derived from plants (e.g. wood or cellulose material) or animals (e.g. animal fat, such as lard, tallow or milk fat), including fungi, yeast, algae and bacteria. Said plants and microbial sources (including yeast and bacteria) may be genemanipulated. Preferably, the renewable feedstock comprises, or is derived from, oil (in particular fatty oil), such as plant or vegetable oil, including wood based oil, animal oil, fish oil, algae oil, and/or microbial oil, fat, such as plant or vegetable fat, animal fat, and/or fish fat, recycled fats of food industry, and/or combinations thereof. The renewable feedstock may comprise, or be derived from, any other origin that can be subjected to biomass gasification or biomass to liquid (BTL) methods.

The renewable feedstock may be subjected to an optional pre-treatment before preparation of a hydrocarbon material, or of a renewable isomeric raw material. Such pre-treatment may comprise purification and/or chemical modification, such as saponification or transesterification. If the renewable raw material is a solid material (at ambient conditions), it is useful to chemically modify the material so as to derive a liquid renewable feedstock. In a preferred embodiment, the renewable feedstock is a liquid renewable feedstock (at ambient conditions).

Preferably, the renewable feedstock is an oxygen-containing feedstock, such as an oil and/or fat. Oil(s) and fat(s) are particularly preferably because these feedstocks have a quite well-defined carbon number length (or distribution) and thus allow good optimization of processing conditions. Preferably, the renewable feedstock comprises at least one of vegetable oil, vegetable fat, animal oil, and animal fat. These materials are particularly preferred, since they allow providing a renewable feedstock having a predictable composition which can be adjusted as needed by appropriate selection and/or blending of the natural oil(s) and/or fat(s).

**Hydrocarbon material**

The isomeric raw material of the present invention may be provided by isomerizing a hydrocarbon material obtained from the renewable feedstock and/or from a renewable source.

Generally, the hydrocarbon material may be produced from the renewable feedstock using any known method. Specific examples of a method for producing the hydrocarbon material are provided in the European patent application EP 1741768 A1. Also other methods may be employed, particularly another BTL (Biomass-To-Liquid) method may be chosen, for example biomass gasification followed by a Fischer-Tropsch method.

In the present invention, it is preferred that the hydrocarbon material is prepared from a renewable feedstock (or source) by a provision step comprising subjecting the renewable feedstock to a deoxygenation treatment (deoxygenation step). This procedure is particularly favourable for a renewable feedstock (or source) having a high oxygen content, such as a feedstock comprising fatty acids, or fatty acid derivatives, such as triglycerides, or a combination thereof.

In the present invention, the deoxygenating method is not particularly limited and any suitable method may be employed. Suitable methods are, for example, hydrotreating, such as hydrodeoxygenation (HDO), catalytic hydrodeoxygenation (catalytic HDO), catalytic cracking (CC), or a combination thereof. Other suitable methods include decarboxylation and decarbonylation reactions, either alone or in combination with hydrotreating. When the deoxygenation method is, for example, catalytic cracking, the cracking conditions may be adjusted such that an isomeric raw material is obtained without the need for an additional isomerization step.

Preferably, the deoxygenation treatment, to which the renewable feedstock is subjected, is hydrotreatment. More preferably, the renewable feedstock is subjected to hydrodeoxygenation (HDO) which preferably uses a HDO catalyst. Catalytic HDO is the most common way of removing oxygen and has been extensively studied and optimized. However, the present invention is not limited thereto. As the HDO catalyst, a HDO catalyst comprising hydrogenation metal supported on a carrier may be used. Examples include a HDO catalyst comprising a hydrogenation metal selected from a group consisting of Pd, Pt, Ni, Co, Mo, Ru, Rh, W or a combination of these. Alumina or silica is suited as a carrier, among others. The hydrodeoxygenation step may, for example, be conducted at a temperature of 100-500 °C and at a pressure of 10-150 bar (absolute).

Preparing a hydrocarbon material from the renewable feedstock may comprise a step of hydrocracking hydrocarbons in the renewable feedstock (after optional hydrotreatment). Thus, the chain length of the hydrocarbon material may be adjusted and the product distribution of the biohydrocarbon mixture obtained by cracking the isomeric raw material (the hydrocarbon material after optional isomerization) can be indirectly controlled.

As in the case of the renewable feedstock, hydrocarbon material may be a blend of materials originating from the renewable source and materials of fossil origin, such as fossil naphtha, but preferably contains at least 20 wt.-% of renewable components, more preferably at least 50 wt.-% or at least 80 wt.-% and may be a fully (100%) renewable hydrocarbon material.

**Isomerization step**

The (renewable) isomeric raw material of the present invention may be provided by subjecting at least straight chain alkanes in a hydrocarbon material to an isomerization treatment to prepare the isomeric raw material. The hydrocarbon material is derived from a renewable feedstock (or source) and is preferably the hydrocarbon material described above.

The isomerization treatment causes branching of hydrocarbon chains, i.e. isomerization, of the hydrocarbon material. The isomeric hydrocarbons, or iso-paraffins, formed by the isomerization treatment may have one or more side chains, or branches. In a preferred embodiment, the formed iso-paraffins have one or more C1 - C9, preferably C1 - C2, branches. Usually, isomerization of the hydrocarbon material produces predominantly methyl branches.

The severity of isomerization conditions and choice of catalyst controls the amount of methyl branches formed and their distance from each other and thus influences the product distribution obtained after thermal cracking. The current inventors have found that the content of iso-paraffins in the isomeric raw material significantly influences the yield of C4 olefins in the thermal cracking step. Providing an isomeric raw material containing at least 60 wt.-% iso-paraffins ensures a good yield of C4-olefins in the cracking product. In addition, the amounts and ratio of monobranched (e.g. monomethyl substituted) iso-paraffins and multiple branched iso-paraffins influences the yield of C4 olefins in the thermal cracking step (to a lesser extend). In other words, providing an isomeric raw material having a high overall iso-paraffins content and at the same time have a high degree of multi-branched iso-paraffins can further increase the yield of C4 olefins and thus the overall efficiency of the present method.

Providing the renewable isomeric raw material preferably comprises subjecting at least a part of the straight chain alkanes (n-paraffins) in the hydrocarbon material to an isomerization treatment, and optionally controlling production of monobranched and multiple branched iso-paraffins, to prepare the isomeric raw material. The straight chain alkanes (or a portion of the straight chain alkanes) may be separated from the remainder of the hydrocarbon material, the separated straight chain alkanes then subjected to isomerization treatment and then optionally re-unified with the remainder of the hydrocarbon material. In an embodiment of the provision step, a portion of the straight chain alkanes is separated from the remainder of the hydrocarbon material, the separated straight chain alkanes are then subjected to isomerization treatment and then re-unified with the remainder of the hydrocarbon material. Alternatively, all of the hydrocarbon material may be subjected to isomerization treatment.

The isomerization step may be carried out in the presence of an isomerization catalyst, and optionally in the presence of hydrogen added to the isomerisation process. Suitable isomerisation catalysts contain a molecular sieve and/or a metal selected from Group VIII of the periodic table and optionally a carrier. Preferably, the isomerization catalyst contains SAPO-11, or SAPO-41, or ZSM-22, or ZSM-23, or fernerite, and Pt, Pd, or Ni, and $Al_2O_3$, or SiOz. Typical isomerization catalysts are, for example, $Pt/SAPO-11/Al_2O_3$, $Pt/ZSM-22/Al_2O_3$, $Pt/ZSM-23/Al_2O_3$, and $Pt/SAPO-II/SiO_2$. The catalysts may be used alone or in combination. The presence of added hydrogen is particularly preferable to reduce catalyst deactivation. The isomerization catalyst is preferably a noble metal bifunctional catalyst, such as Pt-SAPO and/or Pt-ZSM-catalyst, which is used in combination with hydrogen. The isomerization step may be conducted at a temperature of 200-500 °C, preferably 280-400 °C, and at a pressure of 20-150 bar, preferably 30-100 bar (absolute). The isomerization step may comprise further intermediate steps such as a purification step and a fractionation step.

Incidentally, the isomerization treatment is a step which predominantly serves to isomerize the hydrocarbon material. That is, while most thermal or catalytic conversions (such as HDO) result in a minor degree of isomerization (usually less than 5 wt.-%), the isomerization step which may be employed in the present invention is a step which leads to a significant increase in the iso-paraffin content. The isomerization treatment may also be a step comprising controlling the amounts of monobranched and multiple branched iso-paraffins in the prepared isomeric raw material.

It is preferred that the iso-paraffin content (wt.-%) is increased by the isomerization treatment by at least 10 percentage points, more preferably at least 20 percentage points, and even more preferably at least 40 percentage points. More specifically, assuming that the iso-paraffin content of the hydrocarbon material (organic material in the liquid component) is 1 wt.-%, then the iso-paraffin content of the intermediate product after isomerization (e.g. the isomeric raw material) is most preferably at least 85 wt.-% (an increase of 84 percentage points).

Although the isomerization degree is not particularly limited and may reach 100 wt.-%, it is usually more efficient to limit the isomerization degree to 99 wt.-% or less, which is therefore preferred.

The iso-paraffin content can be controlled by the isomerization reaction conditions such as temperature, pressure, residence time and hydrogen content. Moderate isomerization of the hydrocarbon material results in a rather low content of iso-paraffins (about 50 wt.-%), a high number of monobranched iso-paraffins and relatively low content of other branched paraffins. In the present invention, it is therefore preferred to employ more severe isomerization conditions.

Alternatively, or in addition, it is possible to carry out re-isomerization, i.e. to forward all or a part (preferably at least a part containing more than 20 wt.-% n-paraffins) of the effluent of a first isomerization step to a second isomerization step. In this case, the first isomerization step and the second (re-)isomerization step are commonly referred to as "isomerization step".

An isomeric raw material obtained by an isomerization treatment as described above may be fed directly to the thermal cracking procedure. In case n-paraffins have been separated from a hydrocarbon material containing n-paraffins and iso-paraffins, the isomeric raw material obtained by an isomerization treatment (of the n-paraffins material) may be re-unified directly with the remainder of the hydrocarbon material (i.e. the part already having a high iso-paraffin content) and then fed directly to the thermal cracking procedure. That is, no purification is necessary after the isomerization step, so that the efficiency of the process can be further improved.

The hydrotreatment step and the isomerization step may be conducted in the same reactor. Alternatively, hydrotreatment step and the isomerization step may be conducted in separate reactors. Water and light gases, such as carbon monoxide, carbon dioxide, hydrogen, methane, ethane, and propane, may be separated from the hydrotreated or hydrocracked composition and/or from the isomeric raw material with any conventional means, such as distillation, before thermal cracking. After or along with removal of water and light gases, the composition may be fractionated to one or more fractions, each of which may be provided as the isomeric raw material in the thermal cracking step. The fractionation may be conducted by any conventional means, such as distillation. Further, the isomeric raw material may optionally be purified. The purification and/or fractionation allows better control of the properties of the isomeric raw material, and thus the properties of the biohydrocarbon mixture produced in the thermal cracking step.

In the present invention it is preferred that a renewable feedstock comprising at least one of vegetable oil, vegetable fat, animal oil, and animal fat is subjected to hydrotreatment and isomerization, wherein production of monobranched and multiple branched iso-paraffins is controlled during the isomerization treatment, to prepare an isomeric raw material. Preferably, the isomeric raw material comprises at least one of a diesel range fraction (boiling point: 180-360°C, as measured according to EN-ISO-3405 (2011)) and a naphtha range fraction (boiling point: 30-180°C, as measured according to EN-ISO-3405 (2011)). In an embodiment, the isomeric raw material comprises the diesel range fraction. In an alternative embodiment, the isomeric raw material comprises the naphtha range fraction. The isomeric raw material comprising the diesel range fraction and/or the naphtha range fraction is then subjected to thermal cracking, preferably steam cracking. That is, in an embodiment only the diesel range fraction is subjected to thermal cracking, wherein an alternative embodiment comprises subjecting only the naphtha range fraction to thermal cracking. In yet another embodiment, a mixture of the diesel range fraction and the naphtha range fraction is subjected to thermal cracking. Most preferably, the diesel range fraction is subjected to thermal cracking.

Using these fractions, in particular such fractions derived from renewable oil and/or fat, allows good control of the composition of the isomeric raw material, and thus of the biohydrocarbon mixture produced by the cracking step of the invention. Thermally cracking said fraction or fractions gives a desirable product distribution in the thermal cracking step.

## Thermal cracking

Preferably, the thermal cracking of cracking step of the method according to the invention is steam cracking. Steam cracking facilities are widely used in petrochemical industry and the processing conditions are well known, thus requiring only few modifications of established processes. A conventional naphtha (steam) cracker, i.e. a cracker commonly used to thermally crack fossil naphtha, is preferably used to conduct the thermal cracking step. Thermal cracking is preferably carried out without catalyst. However, additives, such as dimethyl disulphide (DMDS), may be used in the cracking step to reduce coke formation.

A good C4 olefin yield can be obtained when performing the thermal cracking step at a COT selected from a wide temperature range. The COT is usually the highest temperature in the cracker. In the present invention, thermally cracking the renewable isomeric raw material is preferably conducted at a coil outlet temperature (COT) selected from the range from 720 °C to 880 °C. Since the yield of C4 olefins tends to drop with higher COT, the COT is preferably 860 °C or lower, more preferably 850 °C or lower, 840 °C or lower, or 830 °C or lower. In order to ensure sufficient cracking, the COT is preferably at least 720 °C, more preferably at least 740 °C, at least 760 °C, at least 780 °C or at least 800 °C.

In a preferred embodiment, the COT is selected from the range from 780 °C to 840 °C. The the COT is even more preferably selected from the range from 800 °C to 830 °C. The thermal cracking may be conducted at a COT of about 820 °C. The COT may, for example, be about 810 °C, 815 °C, 820 °C, 825 °C, or 830 °C. Temperatures selected from the lower part of the above temperature ranges, particularly temperatures below 800 °C, may increase the wt.-% amount of unreacted educts. However, recycling unconverted reactants to the thermal cracking allows a very high overall yield of the process.

The thermal cracking preferably comprises steam cracking. Steam cracking is preferably performed at a flow rate ratio between water and the isomeric raw material ($H_2O$ flow rate [kg/h] / iso-HC flow rate [kg/h]) of 0.05 to 1.20, more preferably 0.05 to 1.10. In a preferred embodiment, the flow rate ratio between water and the isomeric raw material is selected from 0.10 to 1.00. In yet a preferred embodiment, the flow rate ratio between water and the isomeric raw material is selected from 0.20 to 0.80. Even more preferably, the flow rate ratio between water and the isomeric raw material is selected from 0.25 to 0.70. Yet more preferably, the flow rate ratio between water and the isomeric raw material is selected from 0.25 to 0.60. A flow rate ratio selected from the range of 0.30 to 0.50 is particularly favourable, since it allows production of the desired products with high yield. Hence, yet more preferably, the flow rate ratio between water and the isomeric raw material is selected from 0.30 to 0.50.

In general, the coil outlet pressure in the thermal cracking step may be in the range of 0.9 to 3.0 bar (absolute), preferably at least 1.0 bar, more preferable at least 1.1 bar or 1.2 bar, and preferably at most 2.5 bar, more preferably at most 2.2 bar or 2.0 bar.

Preferably, the steam cracking is performed at a flow rate ratio between water and the isomeric raw material ($H_2O$ flow rate [kg/h] / iso-HC flow rate [kg/h]) of 0.30 to 0.50, and at a COT selected from the range from 800 to 820 °C. In a further embodiment, the steam cracking is performed at a flow rate ratio between water and the isomeric raw material ($H_2O$ flow rate [kg/h] / iso-HC flow rate [kg/h]) of 0.30 to 0.50, and at a COT selected from the range from 800 to 840 °C.

**Cracking products**

The term "cracking products" may refer to products obtained directly after a thermal cracking step, or to their derivatives, i.e. "cracking products" as used herein refers to the biohydrocarbon mixture. The cracking product comprises at least C4 olefins in the biohydrocarbon mixture. "Obtained directly after a thermal cracking step" encompasses optional separation and/or purification steps. As used herein, the term "cracking product" may also refer to the biohydrocarbon mixture obtained directly after the thermal cracking step as a whole (i.e. without purification or separation).

The cracking products may include one or more of the following cracking products.

The present invention allows obtaining a biohydrocarbon mixture having a good yield of C4 olefins by thermally cracking the isomeric raw material. C4 olefins, and in particular isobutene, are well suited for the production of petrochemical raw material, in particular as monomers or monomer precursors in polymer industry and as precursors of high-quality drop-in gasoline components.

The cracking products may include one or more of hydrogen, methane, ethane, ethene, propane, propene, propadiene, butane and butylenes, such as butene(s), iso-butene, and butadiene, C5+ hydrocarbons, such as aromatics, benzene, toluene, xylenes, and C5-C18 paraffins and olefins, and their derivatives.

Such derivatives are, for example, methane derivatives, ethene derivatives, propene derivatives, benzene derivatives, toluene derivatives, and xylene derivatives, and their derivatives.

Methane derivatives include, for example, ammonia, methanol, phosgene, hydrogen, oxochemicals and their derivatives, such as methanol derivatives. Methanol derivatives include, for example, methyl methacrylate, polymethyl methacrylate, formaldehyde, phenolic resins, polyurethanes, methyl-tert-butyl ether, and their derivatives.

Ethene derivatives include, for example, ethylene oxide, ethylene dichloride, acetaldehyde, ethylbenzene, alpha-olefins, and polyethylene, and their derivatives, such as ethylene oxide derivatives, ethylbenzene derivatives, and acetaldehyde derivatives. Ethylene oxide derivatives include, for example, ethylene glycols, ethylene glycol ethers, ethylene glycol ethers acetates, polyesters, ethanol amines, ethyl carbonates and their derivatives. Ethylbenzene derivatives include, for example, styrene, acrylonitrile butadiene styrene, styrene-acrylonitrile resin, polystyrene, unsaturated polyesters, and styrene-butadiene rubber, and their derivatives. Acetaldehyde derivatives include, for example, acetic acid, vinyl acetate monomer, polyvinyl acetate polymers, and their derivatives. Ethyl alcohol derivatives include, for example, ethyl amines, ethyl acetate, ethyl acrylate, acrylate elastomers, synthetic rubber, and their derivatives. Further, ethene derivatives include polymers, such as polyvinyl chloride, polyvinyl alcohol, polyester such as polyethylene terephthalate, polyvinyl chloride, polystyrene, and their derivatives.

Propene derivatives include, for example, isopropanol, acrylonitrile, polypropylene, propylene oxide, acrylic acid, allyl chloride, oxoalcohols, cumens, acetone, acrolein, hydroquinone, isopropylphenols, 4-hethylpentene-1, alkylates, butyraldehyde, ethylene-propylene elastomers, and their derivatives. Propylene oxide derivatives include, for example, propylene carbonates, allyl alcohols, isopropanolamines, propylene glycols, glycol ethers, polyether polyols, polyoxypropyleneamines, 1,4-butanediol, and their derivatives. Allyl chloride derivatives include, for example, epichlorohydrin and epoxy resins. Isopropanol derivatives include, for example, acetone, isopropyl acetate, isophorone, methyl methacrylate, polymethyl methacrylate, and their derivatives. Butyraldehyde derivatives include, for example, acrylic acid, acrylic acid esters, isobutanol, isobutylacetate, n-butanol, n-butylacetate, ethylhexanol, and their derivatives. Acrylic acid derivatives include, for example, acrylate esters, polyacrylates and water absorbing polymers, such as super absorbents, and their derivatives.

Butylene derivatives include, for example, alkylates, methyl tert-butyl ether, ethyl tert-butyl ether, polyethylene copolymer, polybutenes, valeraldehyde, 1,2-butylene oxide, propylene, octenes, sec-butyl alcohol, butylene rubber, methyl methacrylate, isobutylenes, polyisobutylenes, substituted phenols, such as p-tert-butylphenol, di-tert-butyl-p-cresol and 2,6-di-tert-butylphenol, polyols, and their derivatives. Other butadiene derivatives may be styrene butylene rubber, polybutadiene, nitrile, polychloroprene, adiponitrile, acrylonitrile butadiene styrene, styrene-butadiene copolymer latexes, styrene block copolymers, styrene-butadiene rubber.

Benzene derivatives include, for example, ethyl benzene, styrene, cumene, phenol, cyclohexane, nitrobenzene, alkylbenzene, maleic anhydride, chlorobenzene, benzene sulphonic acid, biphenyl, hydroquinone, resorcinol, polystyrene, styrene-acrylonitrile resin, styrene-butadiene rubber, acrylonitrile-butadiene-styrene resin, styrene block copolymers, bisphenol A, polycarbonate, methyl diphenyl diisocyanate and their derivatives. Cyclohexane derivatives include, for example, adipic acid, caprolactam and their derivatives. Nitrobenzene derivatives include, for example, aniline, methylene diphenyl diisocyanate, polyisocyanates and polyurethanes. Alkylbenzene derivatives include, for example, linear alkybenzene. Chlorobenzene derivatives include, for example, polysulfone, polyphenylene sulfide, and nitrobenzene. Phenol derivatives include, for example, bisphenol A, phenol form aldehyde resins, cyclohexanone-cyclohexenol mixture (KA-oil), caprolactam, polyamides, alkylphenols, such as p-nonoylphenol and p-dedocylphenol, ortho-xylenol, aryl phosphates, o-cresol, and cyclohexanol. Toluene derivatives include, for example, benzene, xylenes, toluene diisocyanate, benzoic acid, and their derivatives.

Xylene derivatives include, for example, aromatic diacids and anhydrates, such as terephthalic acid, isophthalic acid, and phthalic anhydrate, and phthalic acid, and their derivatives. Derivatives of terephthalic acid include, for example, terephthalic acid esters, such as dimethyl terephthalate, and polyesters, such as polyethylene terephthalate, polytrimethylene terephthalate, polybutylene terephthalate and polyester polyols. Phthalic acid derivatives include, for example, unsaturated polyesters, and PVC plasticizers. Isophthalic acid derivatives include, for example, unsaturated polyesters, polyethylene terephthalate co-polymers, and polyester polyols.

As already mentioned previously, the biohydrocarbon mixture obtained in the cracking step of the present invention are particularly suitable as raw materials for conventional petrochemistry, and in particular polymer industry. Specifically, the biohydrocarbon mixture shows a product distribution which is similar to, and even favourable over, the product distribution obtained from thermal (steam) cracking of conventional (fossil) raw material. Thus, the biohydrocarbon(s) contained in the biohydrocarbon mixture can be added to the known value-added chain while no significant modifications of production processes are required. In effect, it is thus possible to produce for example polymers derived exclusively from renewable material, or feedstock.

The cracking products of the current invention may be used in a wide variety of applications. Such applications are, for example, consumer electronics, composites, automotive, packaging, medical equipment, agrochemicals, coolants, footwear, paper, coatings, adhesives, inks, pharmaceuticals, electric and electronic appliances, sport equipment, disposables, paints, textiles, super absorbents, building and construction, fuels, detergents, furniture, sportwear, solvents, plasticizers and surfactants.

## Reaction step

The reaction step of the present invention is a step of subjecting at least part of the C4 olefins obtained in the cracking step to a reaction so as to produce the renewable component(s). The reaction may comprise reacting the C4 olefins with other components or with themselves.

In a preferred embodiment, at least part of the C4 olefins are reacted to produce drop-in gasoline components.

Alternatively, or in addition, at least part of the C4 olefins may be reacted to produce a monomer (or monomer mixture) for polymer industry or may be directly used to produce a polymer, optionally together with other (renewable or conventional) monomers.

The reaction step may particularly comprise a step of subjecting at least one of the C4 olefins, preferably at least one of 1-butene, (Z)-2-butene and (E)-2-butene, to an alkylation reaction. The alkylation reaction may comprise a reaction between the at least one C4 olefin and a C4 or C5 alkane, preferably an isoalkane. The C4 alkane may preferably be isobutane. The C5 alkane may preferably be isopentane and/or neopentane.

The alkylation reaction particularly preferably comprises a reaction between the at least one of C4 olefin and isobutane to produce isooctane.

The reaction step may further comprise a step of subjecting at least butadiene contained in the C4 olefins to selective hydrogenation to produce a butene (monoene) and employing the thus produced butene as the at least one C4-olefin alone or in admixture with one or more of the other C4 olefins (excluding butadiene).

The reaction step may comprise (alternatively or in addition) a step of subjecting at least a part of isobutene contained in the C4 olefins to a etherification with a C1 to C3 alcohol to produce a C1 to C3 alkyl tert-butyl ether.

The reaction step may comprise a step of subjecting at least a part of isobutene contained in the C4 olefins to a etherification with methanol and/or ethanol to produce methyl t-butyl ether (MTBE) and/or ethyl t-butyl ether (ETBE).

The above-mentioned reactions are particularly preferable for producing drop-in gasoline components having a high octane number. In this respect, the drop-in gasoline component(s) (single component or mixture of components) preferably has/have a PON of at least 90, more preferably at least 95 and even more preferably at least 100.

Moreover, the C4 olefins (including butadiene) may be used as monomers or monomer precursors in polymer industry. For example, the C4 olefins, and in particular isobutene, may be reacted to produce methyl methacrylate, butyl rubber, polyisobutenes, and substituted phenols. Alternatively, or in addition, these C4 olefins may be used for any other purpose commonly known in petrochemistry.

## EXAMPLES

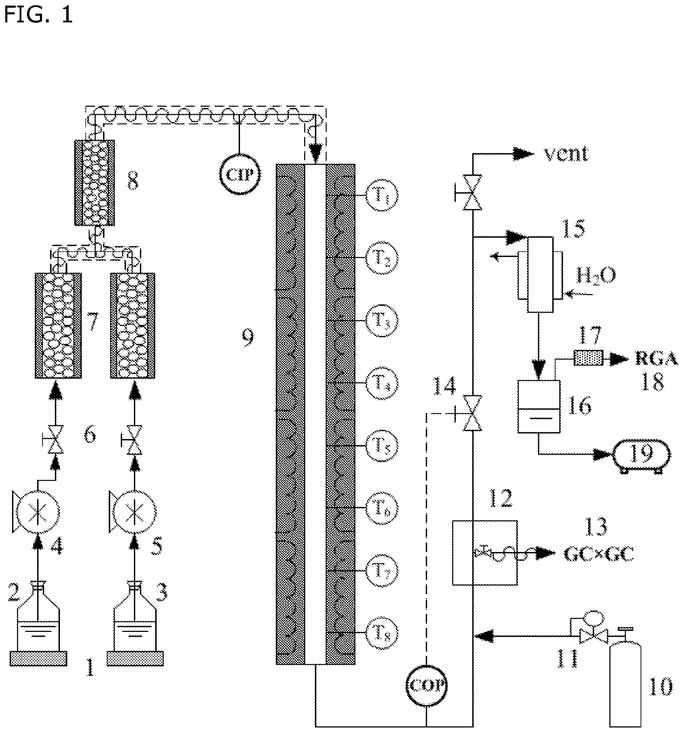

The examples illustrating some embodiments of the current invention were carried out using a laboratory scale equipment shown in Fig. 1

In the laboratory scale equipment of Fig. 1, hydrocarbons and water are provided in reservoir 2 and 3, respectively. Mass flow is determined using an electronic balance 1. Water and hydrocarbons are pumped into evaporators 7 via valves 6 using a water pump 5 and a peristaltic pump 4, respectively. Evaporated materials are mixed in mixer 8 and fed to the reactor 9 having sensors to determine temperatures T1 to T8. Coil inlet pressure (CIP) and coil outlet pressure (COP) are determined using sensors (CIP, COP) at appropriate positions. Reaction products are input into a GC×GC-FID/TOF-MS 13 via a heated sampling oven after having been admixed with an internal standard 10, the addition amount of which is controlled using a coriolis mass flow controller 11. Internal pressure of the reaction system is adjusted using the outlet pressure restriction valve 14. Further, water cooled heat exchanger 15, gas/liquid separator 16, dehydrator 17, refinery gas analyzer 18, and condensate drum 19 are provided to further analyze and recover the products.

MEASUREMENT OF ISOMERIZATION DEGREE

N- paraffin and i-paraffin contents in the renewable isomeric raw material (isomeric raw material) were analyzed by gas chromatography (GC). The samples were analyzed as such, without any pretreatment. The method is suitable for hydrocarbons C2 - C36. N-alkanes and groups of isoalkanes (C1-, C2-, C3-substituted and > C3-substituted) are identified using mass spectrometry and a mixture of known n-alkanes in the range of C2 - C36. The chromatogram is integrated and compounds or compound groups are quantified by normalization using relative response factor of 1.0 to all hydrocarbons. The limit of quantitation for individual compounds was 0.01 wt.-%. Settings of the determination of n- and i-paraffins are shown in Table 1.

Table 1 Settings of GC determination of n- and i-paraffins

|  | GC |
|---|---|
| Injection | split/splitless-injector<br>Split 80:1 (injection volume 0.2 $\mu$L) |
| Column | DB™-5 (length 30m, i.d. 0,25 m, phase thickness 0,25 $\mu$m) |
| Carrier gas | He |
| Detector | FID (flame ionized detector) |
| GC program | 30°C (2min) - 5 °C/min - 300°C (30min), constant flow 1,1 mL/min |

EFFLUENT ANALYSIS

Laboratory scale examples

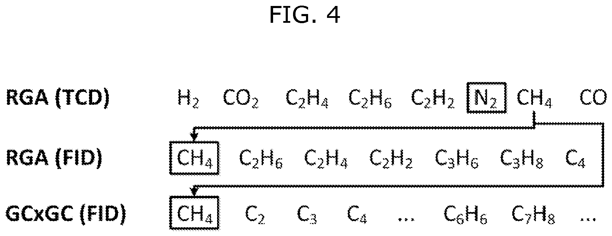

Effluent analysis of the cracking product in the laboratory scale examples, i.e. the examples carried out with the laboratory scale equipment of Fig. 1, was performed using the procedure described by Pyl et.al. (Pyl, S. P.; Schietekat, C. M.; Van Geem, K. M.; Reyniers, M.-F.; Vercammen, J.; Beens, J.; Marin, G. B., Rapeseed oil methyl ester pyrolysis: On-line product analysis using comprehensive two-dimensional gas chromatography. J. Chromatogr. A 2011, 1218, (21), 3217-3223). The quantification of the reactor effluent was done using an external standard ($N_2$) which was added to the reactor effluent in the sampling oven. In order to combine the data of the various instruments, having both thermal conductivity detector (TCD) and flame ionization detector (FID) detectors, multiple reference components were used. This is schematically presented in Figure 4, and described more in detail here below.

The fraction of the reactor effluent containing the permanent gasses and the C4-hydrocarbons was injected on the refinery gas analyzer (RGA). Settings of the RGA are shown in Table 2. $N_2$, $H_2$, CO, $CO_2$, $CH_2$, ethane, ethene and acetylene were detected with a TCD. The mass flow rate of these species, dm/dt, was determined based on the known mass flow rate of the external standard $N_2$ using the following equation, where $A_i$ represents the surface area obtained by the detector. The response factor for each C4-species, f, was determined using a calibration mixture provided by Air Liquide, Belgium.

$$\dot{m}_i = \frac{f_i A_i}{f_{N_2} A_{N_2}} \dot{m}_{N_2}$$

The FID detector on the RGA analyzes C1 to C4 hydrocarbons. Methane, detected on the TCD detector, acted as a secondary internal standard in order to quantify the other detected molecules using the following equation:

$$\dot{m}_i = \frac{f_i A_i}{f_{N_2} A_{CH_4}} \dot{m}_{CH_4}$$

The comprehensive two-dimensional GC, known as GCxGC-FID, allows quantification of the entire effluent stream, aside from $N_2$, $H_2$, CO, COz, and $H_2O$. Methane was used as secondary internal standard. Settings of the GC$\times$GC are shown in Table 3.

Table 2 (refinery gas analyzer settings, laboratory scale examples):

| | | RGA | | |
| --- | --- | --- | --- | --- |
| | | channel 1 | channel 2 | channel 3 |
| Detector | | FID, 200°C | TCD, 160°C | TCD, 160°C |
| Injection (gas) | | 50 µl, 80 °C | 250 µl, 80°C | 250 µl, 80°C |
| Carrier gas | | He | He | $N_2$ |
| Column | Pre Analytical | Rtx™-1[a] | Hayesep™ Q | Hayesep™ T |
| | | Rt™-A1 BOND[b] | Hayesep™ N | Carbosphere™ |
| | | | Molsieve™ 5A | |
| Oven temperature | | 50 → 120°C (5°C/min) | 80°C | 80°C |
| [a]dimethyl polysiloxane (Restek), [b]divinylbenzene ethylene glycol/dimethylacrylate (Restek) | | | | |

Table 3 (GC$\times$GC settings, laboratory scale examples):

| | | GC×GC |
| --- | --- | --- |
| Detectors | | FID, 300°C |
| | | TOF-MS, 35-400 amu |
| Injection | Off-line | 0.2 µl, split flow 150 ml/min, 300°C |
| | on-line | 250µl (gas), split flow 20 ml/min, 300°C |
| Carrier gas | | He |
| Column | First | Rtx™-1 PONA[a] |
| | Second | BPX™-50[b] |
| Oven temperature | Off-line | 40 °C → 250°C (3°C/min) |
| | On-line | -40°C (4 min hold) → 40°C (5°C/min) → 300 °C (4°C/min) |
| Modulation period | | 5s |
| [a]dimethyl polysiloxane (Restek), [b] 50 % phenyl polysilphenylene-siloxane (SGE) | | |

**Isomeric raw material**

**Isomeric raw material composition RC1**

A mixture (isomeric composition) comprising about 53 wt.-% monomethyl substituted iso-paraffins, about 16 wt.-% multi-branched iso-paraffins, and about 31 wt.-% n-paraffins was provided (iso-paraffin content: 69 wt.-%). The composition of the mixture was analyzed by GC analysis and the results are shown in Table 4. The composition corresponds to a hydrocarbon composition (diesel fraction) derived from a renewable feedstock which is subjected to hydrotreating and isomerization.

**Isomeric raw material composition RC2**

A mixture (isomeric composition) comprising about 38 wt.-% monomethyl substituted iso-paraffins, about 55 wt.-% multi-branched iso-paraffins, and about 7 wt.-% n-paraffins was provided (iso-paraffin content: 93 wt.-%). The composition of the mixture was analyzed by GC analysis and the results are shown in Table 4. The composition corresponds to a hydrocarbon composition (diesel fraction) derived from a renewable feedstock which is subjected to hydrotreating and isomerization, but so that a composition having a higher degree (wt.-% amounts) of iso-paraffins than composition RC1 was obtained.

**Isomeric raw material composition RC3**

A mixture (isomeric composition) comprising about 29 wt.-% monomethyl substituted iso-paraffins, about 66 wt.-% multi-branched iso-paraffins, and about 5 wt.-% n-paraffins was provided (iso-paraffin content: 95 wt.-%). The composition of the mixture was analyzed by GC analysis and the results are shown in Table 4. The composition corresponds to a hydrocarbon composition (diesel fraction) derived from a renewable feedstock which is subjected to hydrotreating and isomerization. The isomerization was performed so that a composition having a higher degree (wt.-% amounts) of iso-paraffins than compositions RC1 and RC2 was obtained.

Table 4: Composition of the renewable isomeric diesel samples

| Carbon number | RC1 | | | RC2 | | | RC3 | | |
|---|---|---|---|---|---|---|---|---|---|
| | nP | iP (mono) | iP (multi) | nP | iP (mono) | iP (multi) | nP | iP (mono) | iP (multi) |
| 2 | 0.00 | 0.00 | 0.00 | 0.00 | 0.00 | 0.00 | 0.00 | 0.00 | 0.00 |
| 3 | 0.00 | 0.00 | 0.00 | 0.00 | 0.00 | 0.00 | 0.00 | 0.00 | 0.00 |
| 4 | 0.00 | 0.00 | 0.00 | 0.01 | 0.00 | 0.00 | 0.01 | 0.00 | 0.01 |
| 5 | 0.00 | 0.00 | 0.00 | 0.02 | 0.00 | 0.01 | 0.03 | 0.00 | 0.03 |
| 6 | 0.06 | 0.00 | 0.03 | 0.05 | 0.00 | 0.04 | 0.06 | 0.00 | 0.10 |
| 7 | 0.14 | 0.00 | 0.21 | 0.09 | 0.00 | 0.12 | 0.18 | 0.00 | 0.39 |
| 8 | 0.14 | 0.00 | 0.23 | 0.26 | 0.00 | 0.51 | 0.49 | 0.00 | 1.81 |
| 9 | 0.16 | 0.00 | 0.27 | 0.23 | 0.00 | 0.76 | 0.44 | 0.00 | 2.82 |
| 10 | 0.15 | 0.00 | 0.30 | 0.19 | 0.00 | 0.91 | 0.36 | 0.00 | 3.29 |
| 11 | 0.15 | 0.19 | 0.10 | 0.15 | 0.66 | 0.27 | 0.28 | 0.35 | 1.66 |
| 12 | 0.19 | 0.20 | 0.11 | 0.13 | 0.67 | 0.41 | 0.22 | 1.36 | 3.07 |
| 13 | 0.25 | 0.28 | 0.12 | 0.11 | 0.64 | 0.48 | 0.17 | 1.21 | 2.02 |
| 14 | 0.43 | 0.49 | 0.16 | 0.35 | 0.92 | 0.81 | 0.42 | 1.53 | 2.47 |
| 15 | 5.57 | 6.59 | 1.61 | 1.53 | 5.13 | 4.74 | 1.07 | 5.92 | 6.26 |
| 16 | 9.58 | 15.06 | 3.79 | 1.60 | 11.64 | 14.97 | 0.27 | 5.96 | 10.86 |
| 17 | 5.26 | 10.30 | 2.97 | 1.88 | 7.54 | 7.86 | 0.83 | 8.44 | 12.42 |
| 18 | 8.73 | 19.03 | 5.91 | 0.79 | 10.14 | 21.63 | 0.31 | 4.21 | 17.17 |
| 19 | 0.06 | 0.20 | 0.10 | 0.04 | 0.15 | 0.32 | 0.01 | 0.20 | 0.42 |
| 20 | 0.06 | 0.22 | 0.09 | 0.02 | 0.12 | 0.27 | 0.01 | 0.09 | 0.35 |
| 21 | 0.01 | 0.03 | 0.01 | 0.01 | 0.05 | 0.06 | 0.00 | 0.04 | 0.05 |
| 22 | 0.01 | 0.04 | 0.02 | 0.01 | 0.05 | 0.07 | 0.00 | 0.02 | 0.06 |
| 23 | 0.01 | 0.03 | 0.01 | 0.01 | 0.04 | 0.05 | 0.00 | 0.01 | 0.02 |
| 24 | 0.01 | 0.04 | 0.02 | 0.01 | 0.03 | 0.06 | 0.00 | 0.01 | 0.01 |
| 25 | 0.00 | 0.00 | 0.00 | 0.00 | 0.00 | 0.00 | 0.00 | 0.00 | 0.00 |
| >C25 | 0.28 | 0.00 | 0.28 | 0.39 | 0.00 | 0.00 | 0.00 | 0.00 | 0.15 |
| Total | 30.96 | 52.69 | 16.35 | 7.48 | 37.78 | 54.74 | 5.19 | 29.34 | 65.47 |

In Table 4, iP(mono) denotes monobranched iso-paraffins, iP(multi) denotes multiple branched iso-paraffins, and nP denotes n-paraffins.

COMPOSITION ANALYSIS OF FOSSIL NAPHTHA

The composition of the fossil naphtha samples were analyzed by gas chromatography according to the EN ISO 22854-2016 (ASTM D 6839-2016) method. The method is suitable for analyzing saturated, olefinic, and aromatic hydrocarbons in gasoline fuels. The density of the naphtha samples were analyzed according to the EN-ISO-12185 (2011) method. The boiling point of the naphtha samples were analyzed according to the EN-ISO-3405 (2011) method.

Naphtha N1

Naphtha N1 is a typical fossil light naphtha feedstock for steam crackers. Characteristics of the feedstock N1 are shown in Table 5.

Table 5. Characteristics of the fossil naphtha samples

| Property | | | N1 |
|---|---|---|---|
| Density (kg/m3) | | | 674.2 |
| Boiling point | | | |
| Initial boiling point | IBP(°C) | | 35.7 |
| End point | EP (°C) | | 85.0 |
| Paraffins (vol-%) | | | 81.0 |
| Olefins (vol-%) | | | 0.5 |
| Naphthenes (vol-%) | | | 16.8 |
| Aromatics (vol-%) | | | 1.7 |

**Composition of the fossil naphtha and isomeric raw material blends**

Blends RC1N1, RC2N1 and RC3N1 were prepared by blending fossil naphtha (N1) and the isomeric raw materials (RC1, RC2 and RC3). Table 6 shows the compositions of the prepared blends.

Table 6. Composition of the blends with isomeric raw materials and fossil naphtha

| Blend | Isomeric raw material content | Fossil naphtha N1 content (wt-%) |
|---|---|---|
| RC1N1 | RC1 (75 wt-%) | 25 |
| RC2N1 | RC2 (75 wt-%) | 25 |
| RC3N1 | RC3 (75 wt-%) | 25 |

**Example 1**

Steam cracking was carried out in laboratory scale using composition RC1 at a temperature (coil outlet temperature, COT) of 800°C and a dilution of 0.5 (flow rate ratio of water to composition PC3; water [kg/h] / RC3 [kg/h]) at 1.7 bar (absolute) in a 1.475 m long tubular reactor made of Incoloy 800HT™ steel (30-35 wt.-% Ni, 19-23 wt.-% Cr, >39.5 wt.-% Fe) having an inner diameter of 6 mm. The isomeric raw material flow rate was fixed at 150 g/h. The coil outlet temperature (COT) was measured at a position 1.24 m downstream of the inlet of the reactor, which corresponds to the region having the highest temperature in the reactor.
The product mixture (biohydrocarbon mixture) was analyzed by GC×GC, as mentioned above. The results of the effluent analysis are shown in Table 5.

**Examples 2 to 9**

Steam cracking was carried out similar to Example 1, except for changing the isomeric raw material, COT and dilution, as indicated in Table 7. The product mixtures (biohydrocarbon mixtures) were analyzed by GC×GC, as disclosed above. The results of the effluent analyses are shown in Table 7.

Table 7: Steam cracking conditions and effluent analysis results for examples 1-9

| Example # | 1 | 2 | 3 | 4 | 5 | 6 | 7 | 8 | 9 |
|---|---|---|---|---|---|---|---|---|---|
| Feedstock | RC1 | RC1 | RC1 | RC2 | RC2 | RC2 | RC3 | RC3 | RC3 |
| COT (°C) | 800 | 820 | 840 | 800 | 820 | 840 | 800 | 820 | 840 |
| Dilution (gH2O/g iso-HC) | 0.5 | 0.5 | 0.5 | 0.5 | 0.5 | 0.5 | 0.5 | 0.5 | 0.5 |
| H2 | 0.40 | 0.50 | 0.60 | 0.45 | 0.54 | 0.60 | 0.48 | 0.56 | 0.64 |
| CH4 | 7.99 | 9.75 | 11.00 | 9.37 | 10.80 | 11.74 | 9.93 | 11.45 | 12.74 |
| C2H6 | 28.22 | 32.75 | 34.35 | 27.65 | 29.56 | 30.23 | 26.62 | 28.91 | 30.52 |
| C3H6 | 17.01 | 18.10 | 17.19 | 19.22 | 18.67 | 17.30 | 19.73 | 19.40 | 18.43 |

(continued)

| Example # | 1 | 2 | 3 | 4 | 5 | 6 | 7 | 8 | 9 |
|---|---|---|---|---|---|---|---|---|---|
| i-C4H10 | 0.03 | 0.45 | 0.02 | 0.04 | 0.04 | 0.03 | 0.05 | 0.04 | 0.04 |
| n-C4H10 | 0.13 | 0.10 | 0.08 | 0.11 | 0.09 | 0.07 | 0.12 | 0.10 | 0.08 |
| t-2-C4H8 | 0.53 | 0.48 | 0.44 | 0.64 | 0.61 | 0.51 | 0.71 | 0.68 | 0.60 |
| 1-C4H8 | 4.40 | 3.49 | 2.25 | 4.41 | 3.20 | 2.09 | 4.52 | 3.47 | 2.36 |
| i-C4H8 | 1.63 | 1.59 | 1.43 | 3.22 | 2.96 | 2.48 | 3.94 | 3.62 | 3.15 |
| c-2-C4H8 | 0.41 | 0.41 | 0.37 | 0.56 | 0.51 | 0.41 | 0.64 | 0.57 | 0.47 |
| MeAc | 0.23 | 0.34 | 0.43 | 0.21 | 0.42 | 0.51 | 0.37 | 0.47 | 0.57 |
| 1,3-C4H6 | 5.73 | 6.79 | 6.77 | 6.47 | 6.68 | 6.51 | 6.41 | 6.83 | 6.81 |
| others | 33.31 | 25.26 | 25.07 | 27.64 | 25.93 | 27.51 | 26.48 | 23.89 | 23.58 |
| C4 total | 13.08 | 13.64 | 11.80 | 15.67 | 14.51 | 12.62 | 16.77 | 15.79 | 14.09 |
| C4 olefins | **12.70** | **12.76** | **11.26** | **15.30** | **13.96** | **12.00** | **16.22** | **15.17** | **13.39** |
| C5+ total | 28.73 | 19.96 | 19.88 | 22.63 | 20.62 | 22.50 | 21.22 | 18.40 | 18.10 |

The "C4 olefins" in Table 7 comprise monoenes (monoolefins) as well as butadiene. Note that "t-2" and "c-2" refer to "trans-2" and "cis-2" olefins, i.e. "(E)-2" and "(Z)-2" olefins, respectively, and i-C4H8 refers to isobutene.

Example 10 to 18

Steam cracking was carried out similar to Example 1, except for changing the renewable isomeric paraffin raw material composition to blends of renewable isomeric raw material and fossil naphtha, COT and dilution, as indicated in Table 8. The results of the effluent analyses are shown in Table 8.

Table 8: Steam cracking conditions and effluent analysis results for examples 10-18

| Example # | 10 | 11 | 12 | 13 | 14 | 15 | 16 | 17 | 18 |
|---|---|---|---|---|---|---|---|---|---|
| **Feedstock** | **RC1N1** | **RC1N1** | **RC1N1** | **RC2N1** | **RC2N1** | **RC2N1** | **RC3N1** | **RC3N1** | **RC3N1** |
| **COT (°C)** | **800** | **820** | **840** | **800** | **820** | **840** | **800** | **820** | **840** |
| **Dilution (gH2O/ g iso-HC)** | **0.5** | **0.5** | **0.5** | **0.5** | **0.5** | **0.5** | **0.5** | **0.5** | **0.5** |
| H2 | 0.45 | 0.56 | 0.65 | 0.52 | 0.59 | 0.68 | 0.49 | 0.65 | 0.71 |
| CH4 | 7.71 | 9.24 | 10.19 | 9.18 | 10.46 | 11.20 | 8.96 | 11.18 | 11.86 |
| C2H6 | 27.03 | 31.52 | 33.73 | 27.06 | 27.78 | 30.56 | 23.57 | 29.12 | 30.29 |
| C3H6 | 17.54 | 18.35 | 17.73 | 18.85 | 17.80 | 17.69 | 18.26 | 19.51 | 18.47 |
| i-C4H10 | 0.03 | 0.02 | 0.51 | 0.04 | 0.03 | 0.03 | 0.34 | 0.04 | 0.04 |
| n-C4H10 | 0.15 | 0.12 | 0.09 | 0.14 | 0.11 | 0.09 | 0.15 | 0.12 | 0.10 |
| t-2-C4H8 | 0.49 | 0.36 | 0.47 | 0.64 | 0.60 | 0.54 | 0.71 | 0.68 | 0.60 |
| 1-C4H8 | 4.85 | 2.82 | 3.13 | 4.60 | 3.39 | 2.79 | 4.13 | 3.52 | 2.83 |
| i-C4H8 | 1.99 | 1.95 | 1.68 | 3.22 | 2.92 | 2.66 | 3.63 | 3.52 | 3.12 |
| c-2-C4H8 | 0.46 | 0.47 | 0.39 | 0.56 | 0.51 | 0.45 | 0.62 | 0.56 | 0.48 |
| MeAc | 0.16 | 0.13 | 0.10 | 0.78 | 0.59 | 0.55 | 1.12 | 0.57 | 0.61 |
| 1,3-C4H6 | 5.59 | 6.39 | 6.64 | 5.98 | 6.11 | 6.31 | 5.46 | 6.39 | 6.60 |
| others | 33.54 | 28.06 | 24.69 | 28.44 | 29.11 | 26.44 | 32.55 | 24.13 | 24.32 |

(continued)

| Example # | 10 | 11 | 12 | 13 | 14 | 15 | 16 | 17 | 18 |
|---|---|---|---|---|---|---|---|---|---|
| C4 total | 13.72 | 12.27 | 13.01 | 15.95 | 14.27 | 13.42 | 16.16 | 15.40 | 14.35 |
| C4 olefins | **13.39** | **11.99** | **12.31** | **15.00** | **13.53** | **12.75** | **14.54** | **14.67** | **13.61** |
| C5+ total | 28.15 | 23.72 | 21.14 | 27.50 | 24.20 | 21.57 | 27.41 | 19.01 | 19.25 |

The thus produced butenes (as well as the butadiene after selective hydrogenation to butene) can be forwarded to alkylation or etherification to give e.g. isooctane, MTBE and ETBE which are suitable blending materials for gasoline fuels having properties which do not require specific adaption. In other words, these materials (and others obtainable from the butenes) are suitable as drop-in gasoline fuel components. The butenes can similarly be forwarded to polymer production, after optional further modification to e.g. methyl methacrylate.

**Claims**

1. A method for producing renewable component(s), the method comprising:

   a provision step of providing an isomeric raw material originating from a renewable source, wherein the isomeric raw material contains at least 60 wt.-% iso-paraffins and the iso-paraffins of the isomeric raw material contain more than 30 wt.-% multi-branched iso-paraffins, wherein the amounts of iso-paraffins and multi-branched iso-paraffins are determined relative to the total paraffin content in the isomeric raw material,
   a cracking step of thermally cracking the isomeric raw material to produce a biohydrocarbon mixture containing C4 olefins, and
   a reaction step of reacting at least a part of the C4 olefins to produce the renewable component(s).

2. The method according to claim 1, wherein said renewable component(s) are drop-in gasoline component(s) having a research octane number (RON) of at least 90.

3. The method according to claim 1, wherein said renewable component(s) are bio-monomer(s) or bio-polymer(s), preferably at least one selected from the group consisting of butyl rubber, methyl methacrylate, polymethyl methacrylate, polyisobutylene, substituted phenol, and polybutene.

4. The method according to any one of the preceding claims, wherein the mixture containing C4 olefins contains at least isobutene and the reaction step of reacting at least a part of the C4 olefins is a step of reacting at least a part of the isobutene to produce the renewable component(s).

5. The method according to any one of the preceding claims, wherein the isomeric raw material contains at least 70 wt.-%, preferably at least 75 wt.-%, at least 80 wt.-%, at least 83 wt.-%, at least 85 wt.-%, at least 90 wt.-%, or at least 95 wt.-% iso-paraffins.

6. The method according to any one of the preceding claims, wherein the iso-paraffins contain more than 40 wt.-% multi-branched iso-paraffins.

7. The method according to any one of the preceding claims, wherein the iso-paraffins contain more than 50 wt.-% multi-branched iso-paraffins, even more preferably more than 55 wt.-%, or more than 60 wt.-% multi-branched iso-paraffins.

8. The method according to any one of the preceding claims, wherein the isomeric raw material is a fraction comprising 50 wt.-% or more of C10-C20 hydrocarbons, preferably 75 wt.-% or more, more preferably 90 wt.-% or more of C10-C20 hydrocarbons.

9. The method according to any one of the preceding claims, wherein the provision step comprises an isomerization step of subjecting at least straight chain alkanes in a hydrocarbon material originating from the renewable source to an isomerization treatment to prepare the isomeric raw material; and/or
   wherein the provision step comprises a deoxygenation step of deoxygenating a renewable feedstock originating from the renewable source and optionally a subsequent isomerization step to prepare the isomeric raw material.

10. The method according to any one of the preceding claims, wherein the renewable source comprises at least one of vegetable oil, vegetable fat, animal oil and animal fat and is subjected to hydrotreatment and optionally to isomerization to prepare the isomeric raw material.

11. The method according to any one of the preceding claims, wherein the thermal cracking in the cracking step comprises steam cracking and the steam cracking is preferably performed at a flow rate ratio between water and the isomeric raw material ($H_2O$ flow rate [kg/h] / iso-HC flow rate [kg/h]) of 0.05 to 1.10.

12. The method according to any one of the preceding claims, wherein the biohydrocarbon mixture comprises at least 8.0 wt.-% C4 olefins, relative to all organic components, preferably at least 10.0 wt.-%, preferably at least 12.0 wt.-%, at least 14.0 wt.-%, or at least 15.0 wt.-% C4 olefins, relative to all organic components.

13. The method according to any one of the preceding claims, wherein the reaction step comprises a step of subjecting at least one of the C4 olefins, preferably at least one of 1-butene, (Z)-2-butene and (E)-2-butene, to an alkylation reaction.

14. The method according to claim 13, wherein the alkylation reaction comprises a reaction between the at least one C4 olefin and a C4 or C5 alkane, preferably an isoalkane.

15. The method according to claim 13 or 14, wherein the alkylation reaction comprises a reaction between the at least one of C4 olefin and isobutane to produce isooctane.

16. The method according to any one of claims 13 to 15, wherein the reaction step further comprises a step of subjecting at least butadiene contained in the C4 olefins to selective hydrogenation to produce a butene monoene and employing the thus produced butene as the at least one C4-olefin alone or in admixture with one or more of the other C4 olefins excluding butadiene.

17. The method according to any one of the preceding claims, wherein the reaction step comprises a step of subjecting at least a part of isobutene contained in the C4 olefins to a etherification with a C1 to C3 alcohol to produce a C1 to C3 alkyl tert-butyl ether.

18. The method according to any one of the preceding claims, wherein the reaction step comprises a step of subjecting at least a part of isobutene contained in the C4 olefins to a etherification with methanol and/or ethanol to produce methyl *t*-butyl ether (MTBE) and/or ethyl *t*-butyl ether (ETBE).

**Patentansprüche**

1. Herstellungsverfahren für erneuerbare Komponente(n), wobei das Verfahren umfasst:

einen Bereitstellungsschritt des Bereitstellens eines isomeren Rohmaterials, das aus einer erneuerbaren Quelle stammt, wobei das isomere Rohmaterial mindestens 60 Gew.-% Isoparaffine enthält und die Isoparaffine des isomeren Rohmaterials mehr als 30 Gew.-% mehrfach verzweigte Isoparaffine enthalten, wobei die Mengen an Isoparaffinen und mehrfach verzweigten Isoparaffinen relativ zum Gesamtparaffingehalt im isomeren Rohmaterial bestimmt sind,
einen Crackschritt, bei dem das isomere Rohmaterial thermisch gecrackt wird, um ein Biokohlenwasserstoffgemisch herzustellen, das C4-Olefine enthält, und
einen Reaktionsschritt, bei dem zumindest ein Teil der C4-Olefine umgesetzt wird, um die erneuerbare(n) Komponente(n) herzustellen.

2. Verfahren nach Anspruch 1, wobei die erneuerbare(n) Komponente(n) Drop-in-Benzinkomponente(n) mit einer Research-Oktanzahl (ROZ) von mindestens 90 sind.

3. Verfahren nach Anspruch 1, wobei die erneuerbare(n) Komponente(n) Biomonomer(e) oder Biopolymer(e) sind, vorzugsweise zumindest eines aus der Gruppe bestehend aus Butylkautschuk, Methylmethacrylat, Polymethylmethacrylat, Polyisobutylen, substituiertem Phenol und Polybuten.

4. Verfahren nach einem der vorhergehenden Ansprüche, wobei das Gemisch, das C4-Olefine enthält, zumindest Isobuten enthält und der Reaktionsschritt der Umsetzung mindestens eines Teils der C4-Olefine ein Schritt der Umsetzung mindestens eines Teils des Isobutens ist, um die erneuerbare(n) Komponente(n) herzustellen.

5. Verfahren nach einem der vorhergehenden Ansprüche, wobei das isomere Rohmaterial mindestens 70 Gew.-%, vorzugsweise mindestens 75 Gew.-%, mindestens 80 Gew.-%, mindestens 83 Gew.-%, mindestens 85 Gew.-%, mindestens 90 Gew.-% oder mindestens 95 Gew.-% Isoparaffine enthält.

6. Verfahren nach einem der vorhergehenden Ansprüche, wobei die Isoparaffine mehr als 40 Gew.-% mehrfach verzweigte Isoparaffine enthalten.

7. Verfahren nach einem der vorhergehenden Ansprüche, wobei die Isoparaffine mehr als 50 Gew.-% mehrfach verzweigte Isoparaffine, noch bevorzugter mehr als 55 Gew.-% oder mehr als 60 Gew.-% mehrfach verzweigte Isoparaffine enthalten.

8. Verfahren nach einem der vorhergehenden Ansprüche, wobei das isomere Rohmaterial eine Fraktion ist, die 50 Gew.-% oder mehr C10-C20-Kohlenwasserstoffe, vorzugsweise 75 Gew.-% oder mehr, weiter bevorzugt 90 Gew.-% oder mehr C10-C20-Kohlenwasserstoffe enthält.

9. Verfahren nach einem der vorhergehenden Ansprüche, wobei der Bereitstellungsschritt einen Isomerisierungsschritt umfasst, bei dem zumindest geradkettige Alkane in einem aus der erneuerbaren Quelle stammenden Kohlenwasserstoffmaterial einer Isomerisierungsbehandlung unterzogen werden, um das isomere Rohmaterial herzustellen; und/oder
wobei der Bereitstellungsschritt einen Desoxygenierungsschritt der Desoxygenierung eines aus der erneuerbaren Quelle stammenden erneuerbaren Ausgangsmaterials und gegebenenfalls einen anschließenden Isomerisierungsschritt zur Herstellung des isomeren Rohmaterials umfasst.

10. Verfahren nach einem der vorhergehenden Ansprüche, wobei die erneuerbare Quelle zumindest eines aus Pflanzenöl, Pflanzenfett, tierischem Öl und tierischem Fett umfasst und einem Hydrotreatment und gegebenenfalls einer Isomerisierung unterzogen wird, um das isomere Rohmaterial herzustellen.

11. Verfahren nach einem der vorhergehenden Ansprüche, wobei das thermische Cracken in dem Crackschritt Dampfcracken (Steamcracking) umfasst und das Dampfcracken vorzugsweise bei einem Durchflussverhältnis zwischen Wasser und dem isomeren Rohmaterial ($H_2O$-Durchfluss [kg/h] / iso-HC-Durchfluss [kg/h]) von 0,05 bis 1,10 durchgeführt wird.

12. Verfahren nach einem der vorhergehenden Ansprüche, wobei das Biokohlenwasserstoffgemisch mindestens 8,0 Gew.-% C4-Olefine, bezogen auf alle organischen Komponenten, vorzugsweise mindestens 10,0 Gew.-%, vorzugsweise mindestens 12,0 Gew.-%, mindestens 14,0 Gew.-% oder mindestens 15,0 Gew.-% C4-Olefine, bezogen auf alle organischen Komponenten, enthält.

13. Verfahren nach einem der vorhergehenden Ansprüche, wobei der Reaktionsschritt einen Schritt umfasst, bei dem zumindest eines der C4-Olefine, vorzugsweise zumindest eines aus 1-Buten, (Z)-2-Buten und (E)-2-Buten, einer Alkylierungsreaktion unterzogen wird.

14. Verfahren nach Anspruch 13, wobei die Alkylierungsreaktion eine Reaktion zwischen dem zumindest einen C4-Olefin und einem C4- oder C5-Alkan, vorzugsweise einem Isoalkan, umfasst.

15. Verfahren nach Anspruch 13 oder 14, wobei die Alkylierungsreaktion eine Reaktion zwischen dem zumindest einen C4-Olefin und Isobutan zur Herstellung von Isooctan umfasst.

16. Verfahren nach einem der Ansprüche 13 bis 15, wobei der Reaktionsschritt ferner einen Schritt umfasst, bei dem zumindest Butadien, das in den C4-Olefinen enthalten ist, einer selektiven Hydrierung unterzogen wird, um ein Butenmonoen zu erzeugen, und das so erzeugte Buten als das zumindest eine C4-Olefin allein oder in Mischung mit einem oder mehreren der anderen C4-Olefine mit Ausnahme von Butadien verwendet wird.

17. Verfahren nach einem der vorhergehenden Ansprüche, wobei der Reaktionsschritt einen Schritt umfasst, bei dem zumindest ein Teil des in den C4-Olefinen enthaltenen Isobutens einer Veretherung mit einem C1- bis C3-Alkohol unterzogen wird, um einen C1- bis C3-Alkyl-*tert*-butylether herzustellen.

18. Verfahren nach einem der vorhergehenden Ansprüche, wobei der Reaktionsschritt einen Schritt umfasst, bei dem zumindest ein Teil des in den C4-Olefinen enthaltenen Isobutens einer Veretherung mit Methanol und/oder Ethanol unterzogen wird, um Methyl-*tert*-butylether (MTBE) und/oder Ethyl-*tert*-butylether (ETBE) herzustellen.

**Revendications**

1. Procédé de production de composant(s) renouvelable(s), le procédé comprenant :

   une étape de fourniture consistant à fournir une matière première isomérique provenant d'une source renouvelable, dans laquelle la matière première isomérique contient au moins 60 % en poids d'isoparaffines et les isoparaffines de la matière première isomérique contiennent plus de 30 % en poids d'isoparaffines multiramifiées, les quantités d'isoparaffines et d'isoparaffines multiramifiées étant déterminées par rapport à la teneur totale en paraffines dans la matière première isomérique,
   une étape de craquage consistant à craquer thermiquement la matière première isomérique pour produire un mélange de biohydrocarbures contenant des oléfines C4, et
   une étape de réaction consistant à faire réagir au moins une partie des oléfines C4 pour produire le(s) composant(s) renouvelable(s).

2. Procédé selon la revendication 1, dans lequel le(s) composant(s) renouvelable(s) sont des composant(s) d'essence drop-in ayant un indice d'octane recherche (RON) d'au moins 90.

3. Procédé selon la revendication 1, dans lequel le(s) composant(s) renouvelable(s) sont des bio-monomère(s) ou des bio-polymère(s), de préférence au moins un choisi dans le groupe constitué par le caoutchouc butyle, le méthacrylate de méthyle, le polyméthacrylate de méthyle, le polyisobutylène, le phénol substitué et le polybutène.

4. Procédé selon l'une quelconque des revendications précédentes, dans lequel le mélange contenant des oléfines C4 contient au moins de l'isobutène et l'étape de réaction consistant à faire réagir au moins une partie des oléfines C4 est une étape de faire réagir au moins une partie de l'isobutène pour produire le(s) composant(s) renouvelable(s).

5. Procédé selon l'une quelconque des revendications précédentes, dans lequel la matière première isomérique contient au moins 70 % en poids, préférentiellement au moins 75 % en poids, au moins 80 % en poids, au moins 83 % en poids, au moins 85 % en poids, au moins 90 % en poids ou au moins 95 % en poids d'isoparaffines.

6. Procédé selon l'une quelconque des revendications précédentes, dans lequel les isoparaffines contiennent plus de 40 % en poids d'isoparaffines multiramifiées.

7. Procédé selon l'une quelconque des revendications précédentes, dans lequel les isoparaffines contiennent plus de 50 % en poids d'isoparaffines multiramifiées, encore plus préférentiellement plus de 55 % en poids, ou plus de 60 % en poids d'isoparaffines multiramifiées.

8. Procédé selon l'une quelconque des revendications précédentes, dans lequel la matière première isomérique est une fraction comprenant 50 % en poids ou plus d'hydrocarbures C10-C20, préférentiellement 75 % en poids ou plus, plus préférentiellement 90 % en poids ou plus d'hydrocarbures C10-C20.

9. Procédé selon l'une quelconque des revendications précédentes, dans lequel l'étape de fourniture comprend une étape d'isomérisation consistant à soumettre au moins des alcanes à chaîne droite dans un matériau hydrocarboné provenant de la source renouvelable à un traitement d'isomérisation pour préparer la matière première isomérique ; et/ou
   dans lequel l'étape de fourniture comprend une étape de désoxygénation consistant à désoxygéner une matière première renouvelable provenant de la source renouvelable et éventuellement une étape d'isomérisation ultérieure pour préparer la matière première isomérique.

10. Procédé selon l'une quelconque des revendications précédentes, dans lequel la source renouvelable comprend au moins une parmi une huile végétale, une graisse végétale, une huile animale et une graisse animale et est soumise à un hydrotraitement et éventuellement à une isomérisation pour préparer la matière première isomérique.

**11.** Procédé selon l'une quelconque des revendications précédentes, dans lequel le craquage thermique dans l'étape de craquage comprend le craquage à la vapeur, et le craquage à la vapeur est de préférence effectué à un rapport de débit entre l'eau et la matière première isomérique (débit de $H_2O$ [kg/h] / débit d'iso-HC [kg/h]) de 0,05 à 1,10.

**12.** Procédé selon l'une quelconque des revendications précédentes, dans lequel le mélange de biohydrocarbures comprend au moins 8,0 % en poids d'oléfines C4, par rapport à tout les composants organiques, préférentiellement au moins 10,0 % en poids, préférentiellement au moins 12,0 % en poids, au moins 14,0 % en poids, ou au moins 15,0 % en poids d'oléfines C4, par rapport à tout les composants organiques.

**13.** Procédé selon l'une quelconque des revendications précédentes, dans lequel l'étape de réaction comprend une étape consistant à soumettre au moins une des oléfines C4, de préférence au moins un des 1-butène, (Z)-2-butène et (E)-2-butène, à une réaction d'alkylation.

**14.** Procédé selon la revendication 13, dans lequel la réaction d'alkylation comprend une réaction entre ladite au moins une oléfine C4 et un alcane C4 ou C5, de préférence un isoalcane.

**15.** Procédé selon la revendication 13 ou 14, dans lequel la réaction d'alkylation comprend une réaction entre ladite au moins une des oléfines C4 et l'isobutane pour produire de l'isooctane.

**16.** Procédé selon l'une quelconque des revendications 13 à 15, dans lequel l'étape de réaction comprend en outre une étape consistant à soumettre au moins le butadiène contenu dans les oléfines C4 à une hydrogénation sélective pour produire un monoène de butène et à utiliser le butène ainsi produit comme ladite au moins une oléfine C4 seule ou mélangée avec une ou plusieurs des autres oléfines C4 à l'exclusion du butadiène.

**17.** Procédé selon l'une quelconque des revendications précédentes, dans lequel l'étape de réaction comprend une étape consistant à soumettre au moins une partie de l'isobutène contenu dans les oléfines C4 à une éthérification avec un alcool C1 à C3 pour produire un alkyle *tert*-butyle éther C1 à C3.

**18.** Procédé selon l'une quelconque des revendications précédentes, dans lequel l'étape de réaction comprend une étape consistant à soumettre au moins une partie de l'isobutène contenu dans les oléfines C4 à une éthérification avec du méthanol et/ou de l'éthanol pour produire du méthyl *t*-butyle éther (MTBE) et/ou de l'éthyl *t*-butyle éther (ETBE).

## FIG. 1

# FIG. 2

# FIG. 3

# FIG. 4

| RGA (TCD) | $H_2$ $CO_2$ $C_2H_4$ $C_2H_6$ $C_2H_2$ $\boxed{N_2}$ $CH_4$ $CO$ |

RGA (TCD)  $H_2$  $CO_2$  $C_2H_4$  $C_2H_6$  $C_2H_2$  $\boxed{N_2}$  $CH_4$  $CO$

RGA (FID)  $\boxed{CH_4}$  $C_2H_6$  $C_2H_4$  $C_2H_2$  $C_3H_6$  $C_3H_8$  $C_4$

GCxGC (FID)  $\boxed{CH_4}$  $C_2$  $C_3$  $C_4$  ...  $C_6H_6$  $C_7H_8$  ...

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2643442 B1
- US 20120142982 A1
- US 2018282632 A1
- US 2018179458 A1
- CN 107056570 A

- US 2002087040 A1
- US 2016326079 A1
- US 3816294 A
- EP 1741768 A1

**Non-patent literature cited in the description**

- **ZHIWEI C. et al.** Mechanism of byproducts formation in the isobutane/butene alkylation on HY zeolites. *RSC ADVANCES,* vol. 8 (7), 3392-3398

- **PYL, S. P. ; SCHIETEKAT, C. M. ; VAN GEEM, K. M. ; REYNIERS, M.-F. ; VERCAMMEN, J. ; BEENS, J. ; MARIN, G. B.** Rapeseed oil methyl ester pyrolysis: On-line product analysis using comprehensive two-dimensional gas chromatography. *J. Chromatogr. A,* 2011, vol. 1218 (21), 3217-3223